# EUROPEAN PATENT SPECIFICATION

(11) **EP 3 876 554 B1**
(45) Date of publication and mention of the grant of the patent: **28.05.2025**
(21) Application number: 20160354.5
(22) Date of filing: 02.03.2020
(51) Int. Cl.: H04R 25/00, A61L 2/10

(54) **CHARGING AND DISINFECTING STATION FOR A HEARING DEVICE, AND METHOD OF ITS OPERATION**
LADE- UND DESINFEKTIONSSTATION FÜR EIN HÖRGERÄT UND VERFAHREN ZU DESSEN BETRIEB
STATION DE CHARGEMENT ET DE DÉSINFECTION D'UN DISPOSITIF AUDITIF ET SON PROCÉDÉ DE FONCTIONNEMENT

(43) Date of publication of application: 08.09.2021
(73) Proprietor: Sonova AG, 8712 Stäfa (CH)
(72) Inventor: RIEPENHOFF, Matthias, 8634 Hombrechtikon (CH)

(56) References cited:
- WO-A1-2019/236109
- KR-A- 20110 071 183
- US-A1- 2012 216 418
- US-A1- 2019 299 260

## Description

### TECHNICAL FIELD

This disclosure relates to a charging and disinfecting station for a hearing device, the station comprising a container having an inner volume in which the hearing device is insertable, a charging port provided at the inner volume and configured to charge a battery included in the hearing device, and a radiation source configured to emit radiation into the inner volume, the radiation effective to disinfect the hearing device, according to the preamble of claim 1. The disclosure also relates to a method of charging and disinfecting a hearing device, according to the preamble of claim 9.

### BACKGROUND

Hearing devices are typically used to improve the hearing capability or communication capability of a user, for instance by compensating a hearing loss of a hearingimpaired user, in which case the hearing device is commonly referred to as a hearing instrument such as a hearing aid, or hearing prosthesis. The hearing device may pick up the surrounding sound with a microphone, process the microphone signal thereby taking into account the hearing preferences of the user of the hearing device, and provide the processed sound signal to an output transducer stimulating the user's hearing. The output transducer can be a miniature loudspeaker, commonly referred to as a receiver, for producing a sound in the user's ear canal. As another example, the output transducer can be an electrode array of a cochlear implant producing electric signals stimulating the auditory nerve. A hearing device may also be used to produce a sound in a user's ear canal based on an audio signal which may be communicated by a wire or wirelessly to the hearing device. Hearing devices are often employed in conjunction with communication devices, such as smartphones, for instance when listening to sound data processed by the communication device and/or during a phone conversation operated by the communication device. More recently, communication devices have been integrated with hearing devices such that the hearing devices at least partially comprise the functionality of those communication devices.

During usage, hearing devices are subject to various contamination sources including particles, bacteria, germs, dust, dirt, cerumen, viruses such as a corona-virus, and the like. The contamination can pose a health risk to the user and can also be harmful for numerous electronic components included in the hearing device. The contamination can be aggravated by humidity accumulating in the hearing device. The humidity can be caused, for instance, by sweat produced at a skin in contact with the hearing device, or a condensation of water vapor which may build up on a surface of a housing of the hearing device or enter the housing through an aperture. The humidity can also cause a corrosion of the electronic components further reducing a longevity and reliable performance of the hearing device. It is therefore desirable to regularly provide for a decontamination of the hearing device, which may be combined with a reduction of humidity of the hearing device.

Some hearing device are equipped with a rechargeable battery. International patent publication number WO 2019/236109 A1 discloses a charging and drying station for a hearing device comprising a sanitizing unit, a drying unit, and a charging terminal. The drying unit comprises an air dryer and a thermal dryer allowing to reduce the humidity of the hearing device during charging of the battery via the charging terminal. The sanitizing unit comprises an ultra-violet (UV) generator allowing to disinfect the hearing device. Allowing a simultaneous charging, disinfecting and humidity reduction of the hearing device can be convenient and time-saving.

WO 2019/236109 A1 discloses a charging and drying station for a hearing aid device comprising a main casing with an utility platform and a covering member mounted thereon, a drying arrangement connected to a control circuitry, a control switch provided on the main casing and electrically connected to the control circuitry, and a hearing aid charger accommodated in a receiving cavity of the main casing. A hearing aid device can be disposed on the utility platform for being simultaneously charged by the hearing aid charger and dried by the drying arrangement. The drying arrangement may comprise a sanitizing unit configured as an ultra-violet (UV) generator arranged to generate ultra-violet radiation primarily toward the utility platform for sanitizing the hearing aid device. The user may activate both the drying and sanitizing functions for both of his hearing devices by manually operating the control switch on the main casing.

US20190299260A1 discloses a treatment system including a treatment region through which a substance to be treated by ultraviolet (UV) radiation is moving or flowing or otherwise placed, e.g. by transporting the substance via a conveyor belt or conduit, wherein the treatment region is defined by a set of ultraviolet transparent windows allowing light emitted from two types of ultraviolet sources passing into the treatment region and providing a hermetical isolation of the treatment region from the ambient. The treatment system further comprises a control component which can operate the ultraviolet sources to ensure that the ultraviolet radiation has a predetermined minimum ultraviolet intensity within the treatment region, which may be selected by a user or adjusted based on one or more attributes of the treatment region and/or the substance treated therein, which may include a movement speed, a transparency, and a level of contamination of the substance as determined by a sensor sensitive to fluorescence or visible appearance or chemical composition of the substance. KR 2011 0071183 A discloses a portable hand-held device, such as a smart phone, personal digital assistant (PDA) and MP3 player, which are prone to contaminations mainly due to holding with contaminated hands, wearing in a pocket, or saliva from a mouth when speaking on the phone. The portable device is configured to determine a degree of contamination based on a usage history comprising a call time, a number of button presses and surface contacts detected by a touch sensor, a movement of the portable device detected by a motion sensor, and a heating temperature relative to an ambient temperature. The portable device is further configured to transmit, based on the determined degree of contamination, a control command to a sterilization device performing a sterilization of the portable device, e.g. a light sterilization, a gas sterilization, or an ultrasonic sterilization, and/or calculating a time value for performing the sterilization.

A prolonged exposure of the hearing device to radiation, however, can also be harmful. Some components of a hearing device, such as a housing accommodating the electronic components, an earpiece intended to be inserted into an ear canal, and/or a connector cable provided in between, often comprise organic and/or synthetic polymers which can be attacked by the radiation. In the context of UV radiation, such a phenomenon is also known as UV degradation. It would therefore be desirable to reduce the radiation exposure to a minimum necessary for the decontamination. In particular, in some situations, a required time of the radiation exposure may be shorter than the time required for recharging and/or drying the hearing device. In other situations, the hearing device may be left in the station for a longer time that would be required for the recharging and/or drying and/or decontamination, for instance for convenience reasons or an inability of the user to remove the hearing device immediately afterwards. This leads to a needlessly long exposure of the hearing device to the radiation.

### SUMMARY

It is an object of the present disclosure to avoid at least one of the above mentioned disadvantages and to provide charging and disinfecting station for a hearing device and/or a hearing device charging and disinfecting system and/or a method of operating a charging and disinfecting station allowing to provide a decontamination of the hearing device at an optimized radiation exposure, in particular to reduce a harmful impact on the hearing device by the radiation exposure. It is another object to provide for an increased longevity of a hearing device recurrently subjected to a decontamination. It is a further object to allow a simultaneous charging and decontamination of the hearing device in a user friendly way, in particular such that an active contribution of the user required for an optimization of the radiation exposure can be reduced or avoided.

At least one of these objects can be achieved by a charging and disinfecting station comprising the features of patent claim 1 and/or a method of operating a charging and disinfecting station comprising the features of patent claim 9. Advantageous embodiments of the invention are defined by the dependent claims and the following description.

Accordingly, a charging and disinfecting station for a hearing device comprises a container having an inner volume in which the hearing device is insertable; a charging port provided at the inner volume and configured to charge a battery included in the hearing device; a radiation source configured to emit radiation into the inner volume, the radiation effective to disinfect the hearing device; and a controller configured to determine a parameter indicative of a degree of contamination of the hearing device when the hearing device is inserted into the inner volume, and to control a duration and/or intensity of the radiation depending on the degree of contamination, wherein (1) the controller is configured to determine a state of charge of the battery and to determine the parameter indicative of the degree of contamination based on the state of charge, and/or (2) the charging and disinfecting station further comprises a communication interface communicatively coupled to the controller, wherein the controller is configured to receive data from the hearing device via the communication interface and to determine the parameter indicative of the degree of contamination depending on the received data, the data received from a memory of the hearing device and comprising information indicative of a charging history of the hearing device. By controlling the duration and/or intensity of the radiation depending on the degree of contamination, an exposure of the hearing device to the radiation can be optimized. Optimizing the radiation exposure can reduce harmful impacts and benefit a longevity of the hearing device. The controlling of the radiation by the controller can reduce personal efforts by the user to optimize the radiation exposure.

A hearing device charging and disinfecting system may comprise a hearing device including a chargeable a battery, and the station.

The present disclosure further proposes a method of charging and disinfecting a hearing device, wherein the hearing device is inserted in an inner volume of a container and a battery of the hearing device is charged inside the inner volume, the method comprising emitting radiation into the inner volume, the radiation effective to disinfect the hearing device; determining a parameter indicative of a degree of contamination of the hearing device; and controlling a duration and/or intensity of the radiation depending on the degree of contamination, wherein the method further comprises (1) determining a state of charge of the battery and determining the parameter indicative of the degree of contamination based on the state of charge, and/or (2) receiving data from a memory of the hearing device, the received data comprising information indicative of a charging history of the hearing device, and determining the parameter indicative of the degree of contamination depending on the received data.

Subsequently, additional features of some implementations of the charging and disinfecting station and/or the hearing device charging and disinfecting system and/or the method of charging and disinfecting a hearing device are described. Each of those features can be provided solely or in combination with at least another feature. The features can be correspondingly provided in some implementations of the station and/or the system and/or the method.

In some implementations, the station comprises a communication interface communicatively coupled to the controller, wherein the controller is configured to receive data from the hearing device via the communication interface and to determine the parameter indicative of the degree of contamination depending on the received data. The data may be received from a memory of the hearing device. The received data may comprise information indicative of a usage history and/or a charging history and/or a degree of contamination of the hearing device. The received data may comprise data determined by a sensor of the hearing device. The data determined by the sensor of the hearing device may comprise data received from the memory of the hearing device, in particular data that has been previously stored in the memory of the hearing device. The data determined by the sensor of the hearing device may comprise data determined inside the inner volume and/or outside the inner volume.

In some implementations, the station comprises a dryer configured to reduce humidity inside the inner volume, wherein the controller is configured to control the dryer depending on the degree of contamination. The dryer may comprise a ventilator configured to provide air exchange between the inner volume and an ambient environment outside the inner volume. The dryer may be configured to raise a temperature inside the inner volume.

In some implementations, the station comprises a sensor configured to provide data indicative of the degree of contamination of the hearing device. The sensor may comprise a humidity sensor and/or a pollution sensor and/or a temperature sensor and/or a pressure sensor and/or an optical sensor.

In some implementations, the station comprises a memory configured to store data. The memory may be a non-volatile memory. The stored data may comprise information indicative of a usage history and/or a charging history and/or a degree of contamination of the hearing device. The controller can be configured to determine the parameter indicative of the degree of contamination based on the stored data.

In some implementations, the controller is configured to determine a state of charge of the battery and to determine the parameter indicative of the degree of contamination based on the state of charge.

In some implementations, the hearing device is configured to determine the parameter indicative of the degree of contamination of the hearing device, wherein the controller is configured to receive the parameter from the hearing device. In some implementations, the controller is configured to determine the parameter indicative of the degree of contamination of the hearing device.

The radiation source may be an ultraviolet light source.

### BRIEF DESCRIPTION OF THE DRAWINGS

Reference will now be made in detail to embodiments, examples of which are illustrated in the accompanying drawings. The drawings illustrate various embodiments and are a part of the specification. The illustrated embodiments are merely examples and do not limit the scope of the disclosure. Throughout the drawings, identical or similar reference numbers designate identical or similar elements. In the drawings:
- Fig. 1: schematically illustrates an exemplary hearing device including a chargeable battery, a processor, a sound detector, another sensor, and an output transducer;
- Fig. 2: schematically illustrates a hearing device charging and disinfecting system comprising a hearing device and a charging and disinfecting station for the hearing device; and
- Fig. 3: illustrates an exemplary method of operating a charging and disinfecting station for a hearing device according to principles described herein.

### DETAILED DESCRIPTION OF THE DRAWINGS

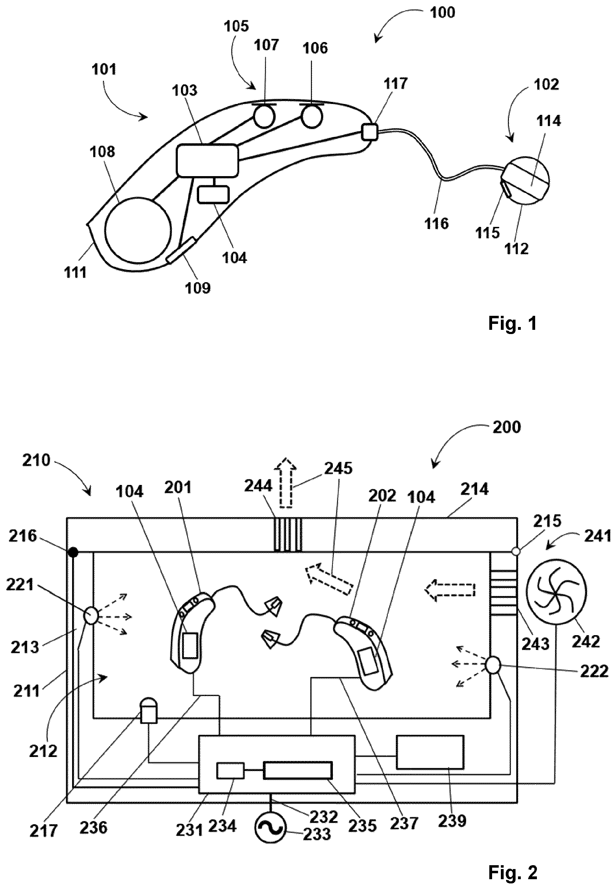

FIG. 1 illustrates an exemplary hearing device 100 configured to be worn at an ear of a user. Hearing device 100 may be implemented by any type of hearing device configured to enable or enhance hearing by a user wearing hearing device 100. For example, hearing device 100 may be implemented as a hearing instrument such as a hearing aid configured to detect sound and to provide an amplified version of the detected sound to a user. Hearing device 100 may also be implemented as a cochlear implant system configured to provide electrical stimulation representative of the detected sound to a user and comprising an implanted part and a removable part prone to contamination, a bimodal hearing system configured to provide both amplification and electrical stimulation representative of the detected sound to a user, or any other suitable hearing prosthesis. In other examples, hearing device 100 may be implemented as an audio playing device, such as an earphone, configured to produce a sound to a user based on an audio signal which may be communicated by a wire or wirelessly to the hearing device. Hearing device 100 may also be implemented as a hearing instrument configured to operate as an audio playing device in an accessory functionality.

Different types of hearing device 100 can also be distinguished by the position at which they are intended to be worn at the ear level of the user. Some types of hearing devices comprise a behind-the-ear part (BTE part) including a housing configured to be worn at a wearing position behind the ear of the user, which can accommodate functional components of the hearing device. Hearing devices with a BTE part can comprise, for instance, receiver-in-the-canal (RIC) hearing aids and behind-the-ear (BTE) hearing aids. Other functional components of such a hearing device may be intended to be worn at a different position at the ear, in particular at least partially inside an ear canal. For instance, a RIC hearing aid may comprise a receiver intended to be worn at least partially inside the ear canal. The receiver may be implemented in a separate housing, for instance an earpiece adapted for an insertion and/or a partial insertion into the ear canal. A BTE hearing aid may further comprise a sound conduit, for instance a sound tube, intended to be worn at least partially inside the ear canal. Other types of hearing devices, for instance earbuds, earphones, and hearing instruments such as in-the-ear (ITE) hearing aids, invisible-in-the-canal (IIC) hearing aids, and completely-in-the-canal (CIC) hearing aids, commonly comprise a housing intended to be worn at a position at the ear such that they are at least partially inserted inside the ear canal.

In the illustrated example, hearing device 100 is implemented as a RIC hearing aid. Hearing aid 100 comprises a BTE part 101 configured to be worn at an ear at a wearing position behind the ear. Hearing aid 100 further comprises an ITE part 102 configured to be worn at the ear at a wearing position at least partially inside an ear canal of the ear. ITE part 102 can be implemented as an earpiece. Hearing aid 100 comprises a first housing 111 of BTE part 101 and a second housing 112 of ITE part 102. First housing 111 accommodates a processor 103 communicatively coupled to a memory 104 and a sound detector 105. In the example, sound detector 105 is provided by a plurality of spatially arranged microphones 106, 107. Microphones 106, 107 can be included in a microphone array. Microphones 106, 107 are configured to provide audio data to processor 103. First housing 111 further accommodates a chargeable battery 108 connected to processor 103. Battery 108 is configured to supply processor 103 and other electronic components connected to processor 103 with energy. A connector 109 is provided at a surface of first housing 111. Processor 103 is communicatively coupled to connector 109. Connector 109 can comprise a charging port configured to be connected to an external energy source in order to recharge battery 108. Connector 109 can comprise a data port configured to be connected to an external data source in order to exchange data. Second housing 112 accommodates an output transducer 114 and a sensor 115. Output transducer 114 may be implemented as a receiver or any other suitable audio output device. BTE part 101 and ITE part 102 are interconnected by a cable 116. Processing unit 103 is communicatively coupled to output transducer 114 and sensor 115 via cable 116 and a cable connector 117 provided at first housing 111 of BTE part 101.

During usage, hearing device 100 is prone to contamination. Hearing device 100 may then be subjected to a radiation source effective to disinfect the hearing device. Various constituent parts of hearing device 100 such as, for instance, first housing 111 and/or second housing 112 and/or cable 116 and/or cable connector 117, are often formed from a material susceptible to the radiation resulting in a degradation of those parts with increasing duration and/or intensity of the radiation. The duration and/or intensity of the radiation may thus be controlled depending on the degree of contamination of the hearing device. To this end, a parameter indicative of the degree of contamination of the hearing device can be determined. These and other operations are described in more detail in the description that follows.

Sensor 115 may be any sensor configured to detect a property on the user and/or in an environment of the hearing device. Sensor data provided by sensor 115 can thus contain information about a usage history and/or a degree of contamination of hearing device 100. For example, sensor 115 may comprise a humidity sensor configured to detect a humidity level inside and/or outside an ear canal. Humidity can nurture a contamination of hearing device 100. An increased humidity level can thus indicate a higher degree of contamination. Sensor 115 may comprise an optical sensor configured to detect light and/or an electrode configured to detect an electric signal induced through a skin of the user. Sensor data comprising information of a decreased sensitivity of the sensor can be indicative of a contamination of hearing device 100. To illustrate, contamination particles accumulating on the optical sensor and/or electrode can cause the decreased sensitivity and can indicate a contamination also occurring on other parts of hearing device 100. Sensor 115 may comprise a biometric sensor configured to measure a biological and/or a physiological characteristic of the user. The biometric sensor may include, for instance, a photoplethysmography (PPG) sensor and/or an electroencephalography (EEG) sensor and/or an electrocardiography (ECG) sensor and/or an electrooculography (EOG) sensor and/or a temperature sensor such as a thermistor, thermopile, thermocouple, solid state sensor, which may be configured to detect a body temperature of the user. Biometric data provided by sensor 115 can comprise information about a usage history of hearing device 100. Sensor 115 may comprise a movement sensor, in particular an inertial sensor such as an accelerometer and/or a gyroscope. Movement data provided by sensor 115 can also comprise information about a usage history of hearing device 100. Sensor 115 may comprise a sound detector, in particular a microphone and/or a voice activity detector (VAD) configured to detect an own voice activity of the user. Sound data provided by sensor 115 can also comprise information about a usage history of hearing device 100.

Memory 104 may be implemented by any suitable type of storage medium and is configured to maintain, e.g. store, data controlled by processor 103, in particular data generated, accessed, modified and/or otherwise used by processor 103. For example, processor 102 may control memory 103 to maintain a data record comprising information about a usage history of hearing device 100 and/or a charging history of battery 108 and/or a degree of contamination of hearing device 100. The usage history may comprise information about a period in which hearing device 100 has been used. This may comprise information about a period in which hearing device 100 was in an operational state and/or a number of times in which hearing device 100 has been turned on by the user. The usage history may also comprise information about a period in which sensor data has been provided by sensor 115, in particular biometric data and/or movement data and/or sound data, and/or by sound detector 105. The charging history of battery 108 may comprise information about a time at which battery 108 has been charged at the last time and/or a charging level to which battery 108 has been charged and/or a duration during which battery 108 has been charged and/or a period between subsequent chargings of battery 108. Processor 103 may be configured to determine a momentary charging level of battery 108. The charging level may be stored in memory 104. The information about a degree of contamination of hearing device 100 can include sensor data provided by sensor 115. For instance, an increased humidity detected by sensor 115 can indicate an increased degree of contamination. A decreased sensitivity of sensor 115, in particular a decline of a quality of the sensor data over time, can also indicate an increased degree of contamination. Processor 103 can be configured to control maintaining of the data record by logging data containing the information over time in memory 104.

FIG. 2 illustrates an exemplary hearing device charging and disinfecting system 200. System 200 comprises a binaural hearing system including a first hearing device 201 configured to be worn at a left ear and a second hearing device 202 configured to be worn at a right ear of the user. In some examples, hearing devices 201, 202 can be implemented corresponding to hearing device 100 illustrated in FIG. 1.

System 200 further comprises a charging and disinfecting station 210 for hearing devices 201, 202. Station 210 comprises a container 211, a radiation source 221, and a controller 231 communicatively coupled to radiation source 221. Container 211 encloses an inner volume 212 in which hearing devices 201, 202 are insertable. Radiation source 221 is provided at inner volume 212 such that it is configured to emit radiation into inner volume 212. Radiation source 221 is a first radiation source. A second radiation source 222 is provided at inner volume 212 such that it is configured to emit radiation into inner volume 212. Multiple radiation sources 212, 222 can allow to provide a more homogenous radiation density inside inner volume 212.

Container 211 comprises a container body 213 and a lid 214. Lid 214 is pivotably mounted to container body 213 by a joint 215. Lid 214 can thus be displaced between different positions including a first position in which container 211 is closed such that inner volume 212 is partially covered by lid 214, and a second position in which container 211 is open such that inner volume 212 is accessible to insert hearing devices 201, 202. A signal generator 216 is operatively coupled to container body 213 and lid 214. Controller 231 is communicatively coupled to signal generator 216. Signal generator 216 is configured to provide a signal indicating whether container 211 is closed or open. For instance, signal generator 216 can be implemented as a contact sensor disposed between container body 213 and lid 214. Controller 231 is configured to control radiation sources 212, 222 to emit the radiation depending on whether container 211 is closed.

Controller 231 comprises a first connector 236 for first hearing device 201 and a second connector 237 for second hearing device 202. Connector 109 of first hearing device 201 is connectable to controller 231 via first hearing device connector 236. Connector 109 of second hearing device 202 is connectable to controller 231 via second hearing device connector 237. Hearing device connector 236, 237 comprises a charging port configured to supply hearing device 201, 202 with energy. Controller 231 comprises a power connector 232 connectable to an external power source 233. Controller 231 is configured to control supplying energy from external power source 233 to hearing device 201, 202 via hearing device connector 236, 237. Hearing device connector 236, 237 can further comprise a communication interface configured to transmit data between hearing device 201, 202 and controller 231. Communication interface 236, 237 can be connectable to a data port of hearing device 201, 202.

Charging and disinfecting station 210 further comprises a contamination sensor 217. Contamination sensor 217 is provided at inner volume 212. Contamination sensor 217 is operative to detect a property inside inner volume 212 indicative of a degree of contamination of hearing device 201, 202 when inserted inside inner volume 212. In some instances, contamination sensor 217 comprises a humidity sensor configured to detect a humidity inside inner volume 212. Increased humidity detected by sensor 217 can indicate an increased degree of contamination of hearing device 201, 202. In some instances, contamination sensor 217 comprises a pollution sensor configured to detect a pollution of inner volume 212. The pollution sensor may be sensitive to gases and/or airborne particles, in particular a density and/or size and/or identity of the particles and/or gases. This may comprise, for instance, biological particles, such as viruses, bacteria and toxins, volatile organic chemicals, hydrocarbons, foreign material in the air and/or the like. Increased pollution detected by sensor 217 can indicate an increased degree of contamination of hearing device 201, 202. In some instances, contamination sensor 217 comprises a temperature sensor configured to detect a temperature inside inner volume 212. Increased temperature detected by sensor 217 can indicate an increased degree of contamination of hearing device 201, 202. In some instances, contamination sensor 217 comprises a pressure sensor configured to detect a pressure inside inner volume 212. Increased pressure detected by sensor 217 can indicate an increased degree of contamination of hearing device 201, 202. The pressure sensor may further comprise a barometric pressure sensor configured to detect a barometric pressure outside inner volume 212 and to compare the pressure inside inner volume with the barometric pressure. In some instances, contamination sensor 217 comprises an optical sensor comprising a light source and a photodetector. Increased light absorption and/or scattering determined by sensor 217 can indicate an increased degree of contamination of hearing device 201, 202. In particular, the optical sensor can be configured to resolve a spectrum of the detected light. The resolved spectrum can allow to identify particles and/or gases representative of the contamination.

Charging and disinfecting station 210 further comprises a dryer 241 configured to reduce humidity inside inner volume 212. Controller 231 is communicatively coupled to dryer 241. In the illustrated example, dryer 241 is an air dryer. Air dryer 241 comprises a ventilator 242 and two ventilation ducts 243, 244 provided in container 211. For instance, as illustrated, first ventilation duct 243 comprises a through hole in a side wall of container body 213 and second ventilation duct 244 comprises a through hole in lid 214. Air dryer 241 can thus provide for a circulation of air between inner volume 212 and an ambient environment outside inner volume 212, as illustrated by dashed arrows 245. In other examples, dryer 241 is a temperature dryer configured to raise a temperature inside inner volume 212 or a combination of an air dryer and a temperature dryer.

Radiation sources 212, 222 can be configured to emit radiation at any wavelength effective to disinfect hearing device 201, 202. For instance, radiation source 212, 222 may comprise an ultra-violet (UV) light source. Controller 231 is configured to control a duration and/or intensity of the radiation emitted by radiation source 212, 222 depending on a degree of contamination of hearing device 201, 202.

Controller 231 comprises a memory 234 and a processor 235. Memory 234 may be implemented by any suitable type of storage medium and is configured to maintain, e.g. store, data controlled by processor 235, in particular data generated, accessed, modified and/or otherwise used by processor 235. For example, processor 235 may control memory 234 to maintain a data record comprising information about a usage history of hearing device 201, 202 and/or a charging history of the battery of hearing device 201, 202 and/or a degree of contamination of hearing device 201, 202. Processor 235 can be configured to control maintaining of the data record by logging data containing the information over time in memory 234. In some instances, the controller 231 can be configured to obtain data containing the information from hearing device 201, 202 via communication interface 236, 237 connected to a data port of hearing device 201, 202. In particular, the data may be stored in memory 104 of hearing device 201, 202, as described above, and transmitted to controller 231 when hearing device 201, 202 is connected to controller 231 by communication interface 236. The data received by controller 231 may then be evaluated by processor 235 and/or stored in memory 234.

Controller 231 is configured to determine the parameter indicative of a degree of contamination of hearing device 201, 202 when the hearing device is inserted into inner volume 212. In some instances, controller 231 is configured to determine the parameter indicative of a degree of contamination of hearing device 201, 202 based on the property detected by sensor 217 inside inner volume 212. In some instances, controller 231 is configured to determine the parameter indicative of the degree of contamination of hearing device 201, 202 based on the data received from hearing device 201, 202 via communication interface 236. In particular, the parameter may be determined based on information about a usage history of hearing device 201, 202 and/or a charging history of battery 108 and/or a degree of contamination of hearing device 201, 202 stored in memory 104 of hearing device 201, 202. To illustrate, a usage history and/or a charging history of battery 108 indicating a longer usage period and/or a previous charging occurring a longer time ago can indicate a larger degree of contamination. The parameter indicative of the degree of contamination of hearing device 201, 202 determined by controller 231 can also be based on a degree of contamination of hearing device 201, 202 determined by a processor of hearing device 201, 202, for instance by evaluating sensor data from sensor 115 as described above.

In some instances, controller 231 is configured to estimate a state of charge of the battery of hearing device 201, 202 and to determine the parameter indicative of the degree of contamination based on the state of charge. To illustrate, a lower state of charge can indicate a longer usage time of hearing device 201, 202 as compared to a higher state of charge which in turn can indicate a larger degree of contamination. The determined state of charge may be stored in memory 234 in order to maintain a data record of the charging history. The determined state of charge may also be compared with a previous state of charge stored in memory 234, for instance a state of charge when hearing device 201, 202 has been previously removed from charging and disinfecting station 210. To illustrate, a difference between a presently determined state of charge and a previous state of charge can indicate a usage time of hearing device 201, 202 indicative of the degree of contamination.

Charging and disinfecting station 210 further comprises a user interface 239. Controller 231 is communicatively coupled to user interface 239. User interface 239 can be configured to provide instructions to controller 231 based on an input of a user. The instructions may comprise a duration and/or intensity of the radiation emitted by radiation source 212, 222. Controller 231 can be configured to prioritize the instructions received from user interface 239 to control the duration and/or intensity of the radiation. User interface 239 can also be configured to provide information to controller 231 based on an input of a user, in particular information about a usage history of hearing device 201, 202 and/or a charging history of the battery of hearing device 201, 202 and/or a degree of contamination of hearing device 201, 202. Controller 231 can be configured to determine the parameter indicative of the degree of contamination of hearing device 201, 202 based on the information received from user interface 239.

In some implementations, controller 231 of hearing device charging and disinfecting system 200 may be provided in hearing device 201, 202. For instance, processor 103 and/or memory 103 of hearing device 201, 202 may be configured to provide the functionality of controller 231 in the above described way when hearing device 201, 202 is inserted into inner volume 212 of container 211. Hearing device 201, 202 may then be connected to power source 233 directly via power connector 232. Hearing device 201, 202 may further be connected to radiation source 212, 222 and/or sensor 217 via a control line connectable to connector 109.

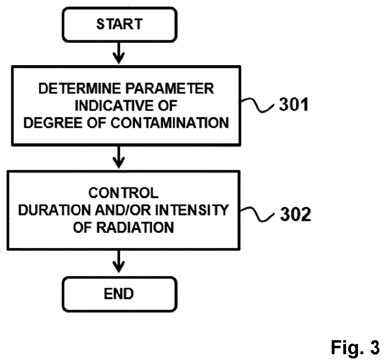

FIG. 3 illustrates a block flow diagram for a method of operating a hearing device. The method may be executed by controller 231. At 301, a parameter indicative of a degree of contamination of hearing device 100, 201, 202 is determined. At 302, a duration and/or intensity of the radiation is controlled depending on the degree of contamination. Determining the parameter indicative of the degree of contamination at 301 may comprise evaluating data comprising information indicative of a usage history and/or a charging history and/or a degree of contamination of the hearing device, and/or receiving sensor data indicative of the degree of contamination, and/or determining a state of charge of the battery of hearing device 100, 201, 202. The data comprising the information may be received from memory 104 of hearing device 100, 201, 202 and/or accessed from memory 234 of controller 231.

While the principles of the disclosure have been described above in connection with specific devices and methods, it is to be clearly understood that this description is made only by way of example and not as limitation on the scope of the invention. The above described preferred embodiments are intended to illustrate the principles of the invention, but not to limit the scope of the invention. Various other embodiments and modifications to those preferred embodiments may be made by those skilled in the art without departing from the scope of the present invention that is solely defined by the claims. In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A charging and disinfecting station for a hearing device, the station comprising
- a container (211) having an inner volume (212) in which the hearing device is insertable;
- a charging port (236, 237) provided at the inner volume (212) and configured to charge a battery (108) included in the hearing device; and
- a radiation source (221, 222) configured to emit radiation into the inner volume (212), the radiation effective to disinfect the hearing device;
**characterized by** a controller (231) configured to
- determine a parameter indicative of a degree of contamination of the hearing device when the hearing device is inserted into the inner volume (212); and to
- control a duration and/or intensity of the radiation depending on the degree of contamination, wherein
the controller (231) is configured to determine a state of charge of the battery (108) and to determine the parameter indicative of the degree of contamination based on the state of charge, and/or
the charging and disinfecting station further comprises a communication interface (236, 237) communicatively coupled to the controller (231), wherein the controller is configured to receive data from the hearing device via the communication interface (236, 237) and to determine the parameter indicative of the degree of contamination depending on the received data, the data received from a memory (104) of the hearing device and comprising information indicative of a charging history of the hearing device.

2. The station according to claim 1, **characterized in that** the radiation source (221, 222) is an ultraviolet light source.

3. The station according to any of the preceding claims, **characterized in that** the received data comprises data determined by a sensor (105, 115) of the hearing device.

4. The station according to claim 3, **characterized in that** the data determined by the sensor (105, 115) of the hearing device comprises data received from the memory (104) of the hearing device.

5. The station according to any of the preceding claims, **characterized by** a dryer (241) configured to reduce humidity inside the inner volume (212), wherein the controller (231) is configured to control the dryer (241) depending on the degree of contamination.

6. The station according to any of the preceding claims, **characterized by** a sensor (217) configured to provide data indicative of the degree of contamination of the hearing device.

7. The station according to claim 6, **characterized in that** the sensor (217) comprises a humidity sensor and/or a pollution sensor and/or a temperature sensor and/or a pressure sensor and/or an optical sensor.

8. The station according to any of the preceding claims, **characterized by** a memory (234) configured to store data, the controller (231) configured to determine the parameter indicative of the degree of contamination based on the stored data.

9. A method of charging and disinfecting a hearing device (100, 201, 202), wherein the hearing device is inserted in an inner volume (212) of a container (211), and a battery (108) of the hearing device is charged inside the inner volume (212), the method comprising
- emitting radiation into the inner volume (212), the radiation effective to disinfect the hearing device (100, 201, 202);
**characterized by**
- determining a parameter indicative of a degree of contamination of the hearing device (100, 201, 202); and
- controlling a duration and/or intensity of the radiation depending on the degree of contamination, wherein the method further comprises
- determining a state of charge of the battery (108) and determining the parameter indicative of the degree of contamination based on the state of charge, and/or
- receiving data from a memory of the hearing device, the received data comprising information indicative of a charging history of the hearing device, and determining the parameter indicative of the degree of contamination depending on the received data.

## Patentansprüche

1. Lade- und Desinfektionsstation für ein Hörgerät, wobei die Station umfasst
- einen Behälter (211) mit einem inneren Volumen (212), in den das Hörgerät eingeführt werden kann;
- einen Ladeanschluss (236, 237), der an dem inneren Volumen (212) bereitgestellt ist und konfiguriert ist, eine Batterie (108), die in dem Hörgerät enthalten ist, zu laden; und
- eine Strahlungsquelle (221, 222), die konfiguriert ist, eine Strahlung in das innere Volumen (212) auszusenden, wobei die Strahlung wirksam ist, das Hörgerät zu desinfizieren;
**gekennzeichnet durch** eine Steuereinheit (231), die konfiguriert ist zum
- Bestimmen eines Parameters, der einen Grad einer Verschmutzung des Hörgeräts angibt, wenn das Hörgerät in das innere Volumen (212) eingeführt ist; und zum
- Steuern einer Dauer und/oder einer Stärke der Strahlung abhängig vom Grad der Verschmutzung, wobei
die Steuereinheit (231) konfiguriert ist, einen Ladezustand der Batterie (108) zu bestimmen und anhand des Ladezustands den Parameter zu bestimmen, der den Grad der Verschmutzung angibt, und/oder
die Lade- und Desinfektionsstation ferner eine Kommunikationsschnittstelle (236, 237) umfasst, die an die Steuereinheit (231) kommunikationstechnisch gekoppelt ist, wobei die Steuereinheit konfiguriert ist, Daten von dem Hörgerät über die Kommunikationsschnittstelle (236, 237) zu empfangen und abhängig von den empfangenen Daten den Parameter zu bestimmen, der den Grad der Verschmutzung angibt, wobei die Daten von einem Speicher (104) des Hörgeräts empfangen werden und Informationen umfassen, die eine Ladehistorie des Hörgeräts angeben.

2. Station nach Anspruch 1, **dadurch gekennzeichnet, dass** die Strahlungsquelle (221, 222) eine Ultraviolettlichtquelle ist.

3. Station nach einem der vorhergehenden Ansprüche, **dadurch gekennzeichnet, dass** die empfangenen Daten Daten umfassen, die durch einen Sensor (105, 115) des Hörgeräts bestimmt werden.

4. Station nach Anspruch 3, **dadurch gekennzeichnet, dass** die durch den Sensor (105, 115) des Hörgeräts bestimmten Daten Daten umfassen, die von dem Speicher (104) des Hörgeräts empfangen werden.

5. Station nach einem der vorhergehenden Ansprüche, **gekennzeichnet durch** einen Trockner (241), der konfiguriert ist, eine Feuchtigkeit innerhalb des inneren Volumens (212) zu reduzieren, wobei die Steuereinheit (231) konfiguriert ist, den Trockner (241) abhängig vom Grad der Verschmutzung zu steuern.

6. Station nach einem der vorhergehenden Ansprüche, **gekennzeichnet durch** einen Sensor (217), der konfiguriert ist, Daten bereitzustellen, die den Grad der Verschmutzung des Hörgeräts angeben.

7. Station nach Anspruch 6, **dadurch gekennzeichnet, dass** der Sensor (217) einen Feuchtigkeitssensor und/oder einen Verschmutzungssensor und/oder einen Temperatursensor und/oder einen Drucksensor und/oder einen optischen Sensor umfasst.

8. Station nach einem der vorhergehenden Ansprüche, **gekennzeichnet durch** einen Speicher (234), der konfiguriert ist, Daten zu speichern, wobei die Steuereinheit (231) konfiguriert ist, anhand der gespeicherten Daten den Parameter zu bestimmen, der den Grad der Verschmutzung angibt.

9. Verfahren zum Laden und Desinfizieren eines Hörgeräts (100, 201, 202), wobei das Hörgerät in ein inneres Volumen (212) eines Behälters (211) eingeführt wird und eine Batterie (108) des Hörgeräts innerhalb des inneren Volumens (212) geladen wird, wobei das Verfahren umfasst
- Aussenden von Strahlung in das innere Volumen (212), wobei die Strahlung wirksam ist, das Hörgerät (100, 201, 202) zu desinfizieren;
**gekennzeichnet durch**
- Bestimmen eines Parameters, der einen Grad der Verschmutzung des Hörgeräts (100, 201, 202) angibt; und
- Steuern einer Dauer und/oder einer Stärke der Strahlung abhängig vom Grad der Verschmutzung, wobei das Verfahren ferner umfasst
- Bestimmen eines Ladezustands der Batterie (108) und Bestimmen anhand des Ladezustands des Parameters, der den Grad der Verschmutzung angibt, und/oder
- Empfangen von Daten von einem Speicher des Hörgeräts, wobei die empfangenen Daten Informationen umfassen, die eine Ladehistorie des Hörgeräts angeben, und Bestimmen des Parameters, der den Grad der Verschmutzung angibt, abhängig von den empfangenen Daten.

## Revendications

1. Station de charge et de désinfection pour un appareil auditif, la station comprenant
- un contenant (211) pourvu d'un volume intérieur (212) dans lequel l'appareil auditif est susceptible d'être introduit ;
- un port de charge (236, 237) situé au niveau du volume intérieur (212) et configuré pour charger une batterie (108) contenue dans l'appareil auditif ; et
- une source de rayonnement (221, 222) configurée pour émettre un rayonnement dans le volume intérieur (212), le rayonnement servant à désinfecter l'appareil auditif ;
**caractérisée par** un contrôleur (231) configuré pour
- déterminer un paramètre indiquant un degré de contamination de l'appareil auditif lorsque l'appareil auditif est introduit dans le volume intérieur (212) ; et pour
- commander une durée et/ou une intensité du rayonnement en fonction du degré de contamination,
le contrôleur (231) étant configuré pour déterminer un état de charge de la batterie (108) et pour déterminer le paramètre indiquant le degré de contamination sur la base de l'état de charge, et/ou
la station de charge et de désinfection comprenant en outre une interface de communication (236, 237) couplée de façon communicative au contrôleur (231), le contrôleur étant configuré pour recevoir des données en provenance de l'appareil auditif via l'interface de communication (236, 237) et pour déterminer le paramètre indiquant le degré de contamination en fonction des données reçues, les données étant reçues en provenance d'une mémoire (104) de l'appareil auditif et comprenant des informations indiquant un historique de charge de l'appareil auditif.

2. Station selon la revendication 1, **caractérisée en ce que** la source de rayonnement (221, 222) est une source de lumière ultraviolette.

3. Station selon l'une quelconque des revendications précédentes, **caractérisée en ce que** les données reçues comprennent des données déterminées par un capteur (105, 115) de l'appareil auditif.

4. Station selon la revendication 3, **caractérisée en ce que** les données déterminées par le capteur (105, 115) de l'appareil auditif comprennent des données reçues en provenance de la mémoire (104) de l'appareil auditif.

5. Station selon l'une quelconque des revendications précédentes, **caractérisée par** un sécheur (241) configuré pour réduire l'humidité à l'intérieur du volume intérieur (212), le contrôleur (231) étant configuré pour commander le sécheur (241) en fonction du degré de contamination.

6. Station selon l'une quelconque des revendications précédentes, **caractérisée par** un capteur (217) configuré pour fournir des données indiquant le degré de contamination de l'appareil auditif.

7. Station selon la revendication 6, **caractérisée en ce que** le capteur (217) comprend un capteur d'humidité et/ou un capteur de pollution et/ou un capteur de température et/ou un capteur de pression et/ou un capteur optique.

8. Station selon l'une quelconque des revendications précédentes, **caractérisée par** une mémoire (234) configurée pour stocker des données, le contrôleur (231) étant configuré pour déterminer le paramètre indiquant le degré de contamination sur la base des données stockées.

9. Procédé de charge et de désinfection pour un appareil auditif (100, 201, 202), l'appareil auditif étant introduit dans un volume intérieur (212) d'un contenant (211), et une batterie (108) de l'appareil auditif étant chargée à l'intérieur du volume intérieur (212), le procédé comprenant
- l'émission d'un rayonnement dans le volume intérieur (212), le rayonnement servant à désinfecter l'appareil auditif (100, 201, 202) ;
**caractérisé par**
- la détermination d'un paramètre indiquant un degré de contamination de l'appareil auditif (100, 201, 202) ; et
- la commande d'une durée et/ou d'une intensité du rayonnement en fonction du degré de contamination, le procédé comprenant en outre
- la détermination d'un état de charge de la batterie (108) et la détermination du paramètre indiquant le degré de contamination sur la base de l'état de charge, et/ou
- la réception de données en provenance d'une mémoire de l'appareil auditif, les données reçues comprenant des informations indiquant un historique de charge de l'appareil auditif, et la détermination du paramètre indiquant le degré de contamination en fonction des données reçues.
